# EUROPEAN PATENT SPECIFICATION

(11) **EP 2 014 318 B1**
(45) Date of publication and mention of the grant of the patent: **26.01.2011**
(21) Application number: 07018176.3
(22) Date of filing: 17.09.2007
(51) Int. Cl.: A61L 27/22, A61L 27/36, A61L 27/50, C07K 14/51

(54) **Method for producing demineralized bone matrix easily releasing bone morphogenetic protein and method for extracting bone morphogenetic protein using demineralized bone matrix by irradiation**
Verfahren zur Herstellung von demineralisierter, leicht knochenmorphogenetisches Protein freisetzender Knochenmatrix und Verfahren zur Extraktion von knochenmorphogenetischem Protein unter Verwendung von demineralisierter Knochenmatrix durch Bestrahlung
Procédé de production de matrice en os déminéralisé avec protéine morphogénétique d'os à libération rapide, et procédé d'extraction de protéine morphogénétique d'os en utilisant la matrice en os déminéralisé par irradiation

(30) Priority: 18.06.2007 KR 20070059657
(43) Date of publication of application: 14.01.2009
(73) Proprietor: Korea Atomic Energy Research Institute, Daejeon 305-353 (KR)
(72) Inventor: Byun, Myung-Woo, Jeongeup-si, Jeollabuk-do 580-050 (KR); Lee, Ju-Woon, Jeongeup-si, Jeollabuk-do 580-050 (KR); Choi, Jong-II, Yeongdeungpo-gu, Seoul 150-782 (KR); Kim, Jae-Hun, Jeongeup-si, Jeollabuk-do 580-753 (KR); Song, Beom-Seok, Jungnang-gu, Seoul (KR); Sung, Nak-Yun, Yeongi-gun, Chungcheongnam-do 339-835 (KR); Lee, Hee-Sub, Hongseong-gun, Chungcheongnam-do 350-851 (KR)
(74) Representative: Zounek, Nikolai

(56) References cited:
- WO-A-96/39203
- WO-A-2005/065396
- US-A1- 2005 136 124

## Description

### BACKGROUND OF THE INVENTION

The present invention relates to a method for producing demineralized bone matrix with improved performance of releasing bone morphogenetic protein by irradiation and a method for extracting bone morphogenetic protein, and more particularly, to a method for producing demineralized bone matrix (hereinafter, often referred to as "bone matrix") with improved performance of releasing bone morphogenetic protein by irradiating the bone matrix, the bone matrix produced by the production method, a method for extracting bone morphogenetic protein using the bone matrix, and the bone morphogenetic protein extracted by the extraction method.

Bone morphogenetic protein (hereinafter often referred to as BMP) belongs to TGFb-super family proteins. As a result of introducing demineralized bone matrix into muscle of a rat, ectopic bone formation was monitored at sites of the muscle containing the bone matrix. From the experiment, it was demonstrated that the bone matrix should contain any material to induce differentiation of undifferentiated cells among cell groups to form the bone in the bone matrix, thereby growing the bone. Such material contained in the bone matrix was a protein ingredient and called "bone morphogenetic protein." For example refer to Urist, MR, Strates, BS, bone morphogenetic protein. J. Dental Res. 50:1392-1406, 1971.

Bone morphogenetic proteins are a differentiation factor and were extracted on grounds of ability to induce the bone formation, as described in Wozney, JM, Science 242:1528-1534, 1988. Such protein produces a BMP family with at least thirty (30) constitutional members belonging to TGFb-super family proteins. The BMP family is classified into sub families including, for example: BMPs such as BMP-2 and BMP-4; osteogenetic proteins (Ops) such as OP-1 or BMP-7, OP-2 or BMP-8, BMP-5, BMP-6 and/or Vgr-1; cartilage derived morphogenetic proteins (CDMPs) such as CDMP-1 or BMP-14 and/or GDF-5; growth/differentiation factors (GDFs) such as GDF-1, GDF-3, GDF-8, GDF-11 or GDF-12 and GDF-14; and other sub families including BMP-3 or osteogenin, BMP-9 or GDF-2, and BMP-10.

Naturally derived bones generally include organic and inorganic materials. The organic materials comprise growth factors, cartilage tissues, collagen and other proteins. The inorganic materials of the bone comprise non-stoichiometrically poorly crystalline apatitic (PCA) calcium phosphate with Ca/P ratio of 1.45 to 1.75, as described in Besic et al., J. Dental Res. 48(1):131, 1969. Mineral ingredients contained in the inorganic materials of the bone are continuously re-absorbed and reproduced by osteoclast and osteoblast in body.

Bone implant is often used to improve natural regeneration of the bone which has defects or injuries. Ideal bone implant must have bio-compatibility, be morphogenetic, that is, osteo-conductive and osteo-inductive at the same time, easily manipulated by a surgeon prior to transplantation, and retain inherent strength and properties in the body after transplantation.

Preferable one of the materials described above is organic bone-derivable materials. Generally known bone-derivable materials include demineralized bone matrix (DBM) and recombined human bone morphogenetic proteins (rh-BMPs). For example refer to: US Patent No. 6,030,635; EP Application No. 0 419 275; International applications PCT/US00/03024, PCT/US99/01677 and PCT/US98/04904, etc. Such organic bone-derivable materials are normally delivered to graft sites together with liquid or gelatin carriers. For example refer to: US Patent Nos. 6,030,635; 5,290,558; 5,073,373; and International application PCT/US98/04904, etc. Ideally used bone implant includes plenty of bone-derivable materials in order to greatly improve the regeneration ability thereof.

WO2005/065396 discloses improved bone matrix compositions and methods for their preparation by exposing demineralized bone matrix to one or more treatments or conditions such as physical, biological or chemical conditions. Certain embodiments refer to the combination of a physical treatment (exposition to radiation) followed by a biological treatment (collagenase) for a better release of bone morphogenetic proteins (BMP). It also describes the addition of protease inhibitors (e.g. N-ethylmaleimide, benzamidine hydrochloride, PMSF) in the biological treatment that do not inhibit collagenase but that inhibit various proteases that digest BMP.

US2005/0136124 discloses methods for high-yield extraction and measuring of osteoinductive agents from demineralized bone matrix. The bone morphogenetic protein BMP-2 is specifically adressed.

Thus there is still a strong requirement for an improved demineralized bone matrix that effectively releases bone morphogenetic proteins in order to develop more efficient bone implant materials, even though development of techniques for efficiently releasing the bone morphogenetic proteins from the bone matrix are as yet insufficient.

Moreover, it is known that irradiation is typically an economical and safe method for hygiene and long term storage of foods and public health products without temperature rise. Gamma radiation is useful in polymer degradation, which cuts bonds which form a molecular polymer by generating free radicals in the bonds. Herein, water molecules function as a catalyst.

Accordingly, it was recently reported that irradiation techniques are applicable for reconstructing and recycling carbonates such as chitosan, alginate, carageenan acid, cellulose, pectin, etc. and, in particular, useful for reducing environmental pollution.

However, it was also disclosed that radiation dose, temperature, contact to atmosphere, storage condition and the like may adversely affect content of nutrients contained in the irradiated foods.

Vitamins suffer some loss when they are treated in amount of more than 1kGy at extremely high temperature and/or in the presence of oxygen. But, extent of the loss is varied depending on kinds of the vitamins and vitamin loss can be generally prevented at low temperature and under anoxic conditions. Minerals show no influence caused by irradiation. Alternatively, carbonates, fats and proteins were demonstrated not to be affected by radiation even at more than 10kGy and only a little affected by irradiation at 50kGy.

### SUMMERY OF THE INVENTION

Accordingly, the present invention is directed to solve the problems of conventional techniques as described above and, an object of the present invention is to provide a method for producing demineralized bone matrix with improved performance of releasing bone morphogenetic protein by irradiating the bone matrix.

It is further an object of the present invention to provide a method for efficiently extracting bone morphogenetic protein by using the demineralized bone matrix with improved performance of releasing the bone morphogenetic protein.

In order to accomplish the above objects, a preferred embodiment of the present invention provides a method for producing demineralized bone matrix with improved performance of releasing bone morphogenetic protein, characterized in that release of the bone morphogenetic protein which constructs the bone matrix is increased by irradiating the bone matrix.

The bone matrix used in extraction of the bone morphogenetic protein according to the present invention can be produced using the demineralized bone matrix derived from mammal and widely known methods and/or techniques. For example refer to Russell et al., Orthopedics, 22(5)524-531, 1999.

Irradiation adopted in the present invention commonly uses at least one selected from a group consisting of gamma ray, electron beam and X-ray and, preferably, uses electron beam in view of improvement of the performance for releasing the bone morphogenetic protein.

Absorption dose of the irradiation ranges from 2.5 to 100kGy and, preferably, from 20 to 50kGy. When the absorption dose is less than 2.5kGy, a desirable purpose of the irradiation is not achieved while there may be problems such as decomposition of materials caused by high dose of radiation, in case that the dose exceeds 100kGy.

Irradiation includes direct irradiation to solid particles in the bone matrix, and irradiation to a composite containing the bone matrix combined with bone restorative carrier.

The above bone restorative carrier includes at least one selected from, for example, carboxymethyl cellulose, chitosan, fibrins and small intestinal submucosa. On the ground that activity for bone morphogenesis is increased during allograft, preferred is carboxymethyl cellulose.

In case of the irradiation to the composite containing the bone matrix combined with the bone restorative carrier, the composite is preferably prepared by combining the bone matrix with the bone restorative carrier in a relative weight ratio ranging from 8:2 to 6:4. The reason is that, in order to use the bone matrix as bio-material for accelerating bone regeneration, the bone matrix should be used as the composite with the bone restorative carrier which is in the form of polymeric gel such as carboxymethyl cellulose in consideration of easier allograft.

The demineralized bone matrix has excellent bone regeneration and can be used in production of bone restorative implants, bone growth accelerating compositions, and/or health aids or supplementary food products.

Another embodiment according to the present invention for achieving the object provides a method for efficiently extracting bone morphogenetic protein by using the demineralized bone matrix with improved performance of releasing bone morphogenetic protein, characterized in that it includes a process of decomposing collagen to form the bone matrix by adding collagenase and salt to the bone matrix with improved performance of releasing the bone morphogenetic protein.

The extraction method of the present invention may additionally include a process of removing salt from a solution obtained by the collagen decomposition process described above through dialysis, that is, after collagen was decomposed by adding collagenase and salt to the demineralized bone matrix with improved performance of releasing the bone morphogenetic protein.

Such salt includes MgCl₂, CaCl₂, NaCl, N-ethylmaleimide (NEM), phenylmethylsulfonyl fluoride (PMSF), benzamidine-HCl and so on, but is not restricted thereto so far as the salt fulfills a function of protease inhibitor and collagenase activation.

The bone morphogenetic protein extracted according to the present invention is not particularly limited, but, preferably BMP-2 or BMP-7.

BMP-2, that is, bone morphogenetic protein-2 strongly derives autologous and heterologous bone morphogenesis *in vivo* and, in addition to, is widely known as an effective bone morphogenetic derivative to differentiate preosteoblast or undifferentiated stem cells into osteoblast *in vitro.* Further, as ectopic bone is formed when BMP-2 is intramuscularly introduced *in vivo,* it was found that, if BMP-2 is introduced into C2C12 cells which are mouse premyoblastic cell lines, such cells stop differentiation into muscle cells but express marker genes of osteoblast.

It is well known that BMP-7 is a material concerning to bone morphogenesis and has an important role of forming teeth and eyes during creation. Moreover, it was disclosed that BMP-7 cannot be generated in body of an adult person. For example refer to Dev. Biol. 207(1): 176-188, 1999.

As described above, the extracted bone morphogenetic protein according to the present invention can be efficiently used in industrial applications such as bone restorative implants, bone growth accelerating compositions and/or health aids or supplementary food products.

### BRIEF DESCRIPTION OF THE DRAWINGS

The above objects, features and advantages of the present invention will become more apparent to those skilled in the related art in conjunction with the accompanying drawing.

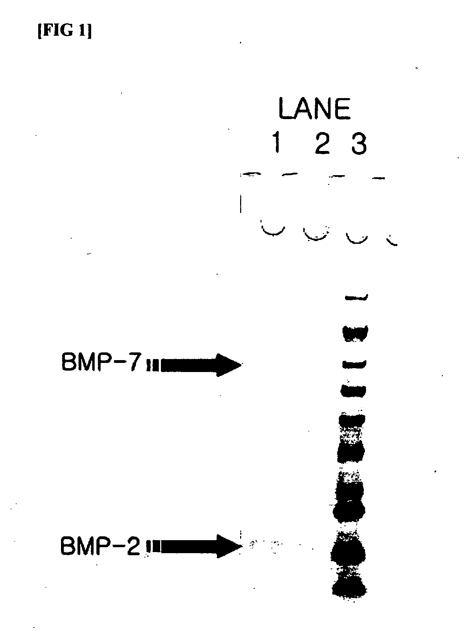

Fig. 1 shows a result represented by comparing amount of BMP-2 and BMP-7 which are the bone morphogenetic proteins extracted from the demineralized bone matrix after irradiation, with desired amount of BMP-2 and BMP-7 which are the bone morphogenetic proteins extracted from a control, that is, the bone matrix without irradiation.

### DETAILED DESCRIPTION OF THE INVENTION

Hereinafter, the present invention will become apparent from the following examples with reference to the accompanying drawings. However, the examples are intended to illustrate the invention as preferred embodiments of the present invention and do not limit the scope of the present invention.

### EXAMPLE 1

### Preparation of demineralized bone matrix with improved performance of releasing bone morphogenetic protein

In this example, the demineralized bone matrix derived from bovine was used. The bone matrix was prepared by the conventional method disclosed in Russell et al., Orthopedics 22(5)524-531, 1999. In order to prepare a composite comprising the bone matrix combined with carboxymethyl cellulose as the bone restorative carrier, carboxymethyl cellulose and the bone matrix were combined together in a relative weight ratio of about 7:3 to form the composite.

The composite consisting of the bone matrix and the bone restorative carrier was subjected to irradiation using a linear electron accelerator of Research Institution of Radiological Science in Jeongeup city, Korea Atomic Energy Research Institute (KAERI). Such accelerator was UELV-10-10S model with electron beam energy of 10MeV and current of 1mA manufactured by NIIEFA, which had an inspection window with distance of 200mm and dimension of 8 × 20mm.

Absorbed dose of the electron beam was determined from current value and radiation dose measured. The radiation dose used was 30kGy.

### EXAMPLE 2

### Extraction of bone morphogenetic protein from demineralized bone matrix

6.5ml of 0.2M tris-HCl buffer solution (pH 7.2) was added to either of 1g of the demineralized bone matrix with irradiation or the same amount of a control, that is, the demineralized bone matrix without irradiation. The mixture was reacted for 2 hours by using a shaking water bath at 37°C.

Thereafter, in order to remove collagen comprising the bone matrix, additional ingredients such as collagenase with final concentration of 100 CDU/ml, 3mM MgCl₂, 3mM CaCl₂, 20mM NaCl, 3mM N-ethylmaleimide (NEM), 0.1mM phenylmethylsulfonyl fluoride (PMSF) and 0.1mM benzamidine-HCl were introduced into the mixture, followed by reaction thereof for more than 16 hours in the shaking water bath at 37°C.

After the reaction, the reacted solution underwent centrifugation at 4000rpm for 20 minutes, then, supernatant of the centrifuged solution was transferred into a tube and underwent dialysis at 4°C for more than 16 hours in order to remove salts from the reacted solution.

After the dialysis, lyophilizing the prepared solution resulted in a protein, which was then dissolved in 10ml oftris-HCl solution (pH 7.4) and stored in frozen condition at -20°C.

### EXAMPLE 3

### Quantification of bone morphogenetic protein

For quantification of the bone morphogenetic protein extracted from the demineralized bone matrix, intensities of proteins BMP-2 and BMP-7 were compared to each other by employing BCA assays (Bicinchoninic acid protein assays) and SDS-PAGE. Alternatively, osteoinductivity of the bone matrix by the bone morphogenetic protein underwent the quantification by ALP assays directly using C2C12 cells.

Details of the BCA assays were complied with instructions in relation to BCA protein assay kit, Sigma Inc.

Several protein samples were prepared from purified BSA (bovine serum albumin) diluted in 1 × PBS (phosphate buffered saline) in concentrations of: 2,000µg/ml; 1,500µg/ml; 1,000µg/ml; 750µg/ml; 500µg/ml; 250µg/ml; 125µg/ml; and 25µg/ml, respectively. 25 µl of the protein sample was mixed with 200µl of BCA working solution and reacted at 37°C for 30 minutes for color expression. Herein, absorbency was determined at A595 using GENYTH 3100 manufactured by ANTHOS.

SDS-PAGE electrophoresis of protein will be described below.

First, A gel is casted on a glass plate according to a typical process for using the electrophoresis kit. Then, stacking gel and running gel solutions were prepared. For mini-gel with dimension of 10 × 8cm and thickness of 0.75mm, it required 4ml and 0.6ml of the running gel and the stacking gel, respectively.

To the running gel solution, were added ammonium persulfate and TEMED (N,N,N',N'-tetramethylethylenediamine; N,N,N',N'-Di-(dimethyamino)ethane; and N,N,N',N'-tetramethyl-1-,2-diaminomethane) which were then rapidly blended to form a mixture without foaming. Subsequently, the mixture was gently poured into a space between the glass plates by means of pipette while considering height of the stacking gel. Distilled water or alcohol was slowly poured over the mixture, followed by leaving the prepared material until the gel cured. When the gel was cured, a barrier between alcohol added to top layer and acrylamide was clearly monitored. After removing the alcohol moiety, the gel was washed several times with the distilled water.

The prepared stacking gel solution was admixed with ammonium persulfate and TEMED, rapidly blended, and gently poured into the space between the glass plates by using the pipette. A comb was fitted into the gel solution.

After the gel was completely cured, the comb was gently put out of the cured gel to form wells in the gel. The wells were rinsed several times with the distilled water. The cast gel was mounted on an electrophoresis apparatus, a buffer solution for the electrophoresis was poured into each of tanks at top and bottom sides, and the prepared sample was carefully applied to the wells.

An electrophoresis tool was connected to a power source to carry out the electrophoresis at constant voltage or current. When bromophenol blue reached an end part of the gel as a tracking marker, the power source was switched off and the glass plate was taken off the electrophoresis apparatus.

After completing the electrophoresis, the gel was carefully separated from the gel plate and transferred to a plastic or glass container. A staining solution was poured into the container and the container was placed on a shaker to stain the gel for 30 minutes to 1 hour. After that, the stained gel was washed with tap water and a bleaching reagent was poured thereto. The treated gel was positioned on the shaker and the bleaching reagent was changed several times to completely remove the staining solution from the gel.

Furthermore, ALP assay directly using C2C12 cells was adopted to quantify osteoinductivity by the bone morphogenetic protein. This assay will be described in detail below.

First, C2C12 cells were added at 5 × 10⁴ cells/well to 24-well plate.

4 hours after adding the cells to the 24-well plate, the media was changed to 1% FBS media and a transwell was placed in the 24-well plate to treat 100mg of the demineralized bone matrix while introducing 1ml of the media thereto.

After culturing for 48 hours, the media were discarded and the cultured cells were rinsed out twice with a cold PBS (phosphate buffered saline). Subsequently, 0.5% triton-100/PBS was added in amount of about 500µℓ to 1 mℓ into the wells and left for 1 to 2 minutes. Then, a scraper was used to scratch the cells off the wells and a freezing/thawing process, that is, lyophilization was repeated three times to break cell membrane.

After dilution in series, the samples were placed into the plates in amount of 50 µℓ per plate. Only the enzyme buffer was introduced in blank of each of the plates, 50 µℓ of pNPP (para-nitrophenyl phosphate) substrate solution was added thereto, and the sample was cultured at room temperature for 10 to 20 minutes.

Finally, after 50µℓ of stop solution was added to the cultured sample and rapidly agitated to blend it, absorbency of the sample was detected at 405nm. An assay buffer was used as standard for the detection, diluted in series and detected at 405nm as was the sample.

Measured values from the experiments were subjected to ANOVA (analysis of variance) using SPSS software and, if they passed the significance test, a significant difference between least square mean values was identified through multiple range tests by Duncan (p < 0.05).

### EXPERIMENTAL EXAMPLE 1

### Comparison of performances of releasing bone morphogenetic proteins of demineralized bone matrices with and without irradiation

The performance of releasing the bone morphogenetic protein of the demineralized bone matrix produced by irradiation as described in Example 1 was compared with that of the control which was the demineralized bone matrix without irradiation.

The results are set out in Table 1 below and, as shown in Table 1, total amount of protein moiety was increased through irradiation by 10% more than that of the control without irradiation in BCA protein test.

**TABLE 1: Comparison of total amounts of bone morphogenetic proteins with and without irradiation**

| Sample | O.D (562nm) | protein(µg/mℓ) | % |
|---|---|---|---|
| Bone matrix protein (0kGy) | 0.262 | 158.27 | 100 |
| Bone matrix protein (30kGy) | 0.28 | 174.64 | 110.3 |

Moreover, as shown in Fig. 1, it was also identified on SDS-PAGE gel that both of the proteins BMP-2 and BMP-7 have improved concentration. In Fig. 1, lane 1 represented BMP-2 and BMP-7 extracted from the demineralized bone matrix with irradiation, lane 2 represented BMP-2 and BMP-7 extracted from the demineralized bone matrix without irradiation, and lane 3 represented protein size markers.

Additionally, ALP test using C2C12 cells from the demineralized bone matrix also demonstrated that potency of the bone matrix with irradiation was increased, as shown in Table 2 below.

**TABLE 2: Comparison of ALP activities of demineralized bone matrices_with and without irradiation**

| | 1 × ALP concentration (pmoles) % |
|---|---|
| DBM 0kGy | 100 |
| DBM 30kGy | 106.42 |

### EXPERIMENTAL EXAMPLE 2

### Comparison of performances of releasing bone morphogenetic protein of demineralized bone matrices combined with carboxymethyl cellulose carriers with and without irradiation

By ALP assay using C2C12 cells, the performances of releasing the bone morphogenetic protein of the demineralized bone matrices with and without irradiation were compared, after combining the bone matrices with carboxymethyl cellulose based carriers in a relative weight ratio of 7:3.

As shown in the following Table 3, it was determined from ALP assay that the bone matrix combined with the carboxymethyl cellulose carrier after irradiation also exhibited increase of activity.

**TABLE 3: Comparison of ALP activities of demineralized bone matrices combined with carboxymethyl cellulose with and without irradiation**

| | 1 × ALP concentration (pmoles) % |
|---|---|
| DBM 0kGy | 100 |
| DBM 30kGy | 118.5 |

As described above, the present invention can produce demineralized bone matrix with improved performance of releasing bone morphogenetic protein by irradiating the bone matrix. And the present invention can extract the bone morphogenetic protein with high efficiency by using the bone matrix.

The bone matrix produced by the above method according to the present invention and the bone morphogenetic protein extracted as described above can be advantageously used in production of, for example, bone restorative implants, bone growth accelerating compositions, and/or health aids or supplementary food products.

## Claims

1. A method for producing demineralized bone matrix with improved performance of releasing bone morphogenetic protein, comprising: irradiating the bone matrix so that the absorption dose of the irradiation is in the range of from 20 to 50kGy.

2. The method according to Claim 1, wherein the irradiation is conducted by at least one selected from a group consisting of electron beam, gamma-ray and X-ray.

3. The method according to Claim 1, wherein the irradiation includes direct irradiation to solid particles in the bone matrix or irradiation to a composite containing the bone matrix combined with bone restorative carrier.

4. The method according to Claim 3, wherein the bone restorative carrier comprises at least one selected from a group consisting of carboxymethyl cellulose, chitosan, fibrins and small intestinal submucosa.

5. The method according to Claim 3, wherein the bone matrix and the bone restorative carrier are combined together in a weight ratio ranging from 8.2 to 6.4

6. The method according to Claim 1, wherein the bone morphogenetic protein is BMP-2 or BMP-7.

7. A method for extracting bone morphogenetic protein by using the demineralized bone matrix obtainable by any one of the methods according to Claims 1 to 6, comprising: addition of collagenase and at least one selected from a group consisting of MgCl₂, CaC1₂, NaCI, N-ethylmaleimide, phenylmethylsulfonyl fluoride and benzamidine-HCL to the bone matrix to decompose collagen contained in the bone matrix.

8. The method according to Claim 7, further comprising removal of the salt contained in the reacted solution through dialysis after decomposition of the collagen.

## Patentansprüche

1. Ein Verfahren zur Herstellung von demineralisierter Knochenmatrix mit verbesserten Eigenschaften bei der Freisetzung von knochenmorphogenetischem Protein, umfassend: Bestrahlung der Knochenmatrix, so dass die Absorptionsdosis der Bestrahlung im Bereich von 20 bis 50 kGy liegt.

2. Das Verfahren nach Anspruch 1, worin die Bestrahlung ausgeführt wird durch mindestens einen ausgewählt aus einer Gruppe bestehend aus Elektronenstrahl, Gammastrahl und Röntgenstrahl.

3. Das Verfahren nach Anspruch 1, worin die Bestrahlung direkte Bestrahlung von Feststoffteilchen in der Knochenmatrix oder Bestrahlung einer Zusammensetzung, enthaltend Knochenmatrix kombiniert mit knochenstärkendem Träger beinhaltet.

4. Das Verfahren nach Anspruch 3, worin der knochenstärkende Träger mindestens eins ausgewählt aus einer Gruppe bestehend aus Carboxymethylcellulose, Chitosan, Fibrinen und Dünndarm-Unterschleimhaut (Submucosa) umfasst.

5. Das Verfahren nach Anspruch 3, worin die Knochenmatrix und der knochenstärkende Träger in einem von 8:2 bis 6:4 reichenden Gewichtsverhältnis zusammen kombiniert werden.

6. Das Verfahren nach Anspruch 1, worin das knochenmorphogenetische Protein BMP-2 oder BMP-7 ist.

7. Ein Verfahren zum Extrahieren knochenmorphogenetischen Proteins durch Verwendung der demineralisierten Knochenmatrix erhältlich nach jedem der Verfahren nach den Ansprüchen 1 bis 6, umfassend: Zugabe von Collagenase und mindestens einem ausgewählt aus einer Gruppe bestehend aus MgCl₂, CaCl₂, NaCl, N-ethylmaleinsäureimid, Phenylmethylsulfonylfluorid und Benzamidin-HCL zur Knochenmatrix, um in der Knochenmatrix enthaltenes Collagen abzubauen.

8. Das Verfahren nach Anspruch 7, des weiteren das Entfernen des in der reagierten Lösung enthaltenen Salzes durch Dialyse nach Abbau des Collagens umfassend.

## Revendications

1. Procédé de production d'une matrice osseuse déminéralisée qui présente une performance améliorée en termes de libération de protéine morphogénétique osseuse, comprenant : l'irradiation de la matrice osseuse de sorte que la dose d'absorption de l'irradiation se situe dans la plage de 20 à 50 kGy.

2. Procédé selon la revendication 1, dans lequel l'irradiation est conduite par au moins un élément choisi dans un groupe constitué par un faisceau d'électrons, un rayon gamma et un rayon X.

3. Procédé selon la revendication 1, dans lequel l'irradiation inclut une irradiation directe de particules solides dans la matrice osseuse ou une irradiation d'un composite contenant la matrice osseuse combinée à un support de restauration osseuse.

4. Procédé selon la revendication 3, dans lequel le support de restauration osseuse comprend au moins un élément choisi dans un groupe constitué par la carboxyméthyl cellulose, le chitosane, les fibrines et la sous-muqueuse de l'intestin grêle.

5. Procédé selon la revendication 3, dans lequel la matrice osseuse et le support de restauration osseuse sont combinés ensemble dans un rapport en poids variant de 8:2 à 6:4.

6. Procédé selon la revendication 1, dans lequel la protéine morphogénétique osseuse et BMP-2 ou BMP-7.

7. Procédé d'extraction d'une protéine morphogénétique osseuse à l'aide de la matrice osseuse déminéralisée pouvant être obtenue par l'un quelconque des procédés selon les revendications 1 à 6, comprenant : l'addition de collagénase et d'au moins un élément choisi dans un groupe constitué par MgCl₂, CaCl₂, NaCI, le N-éthylmaléimide, le fluorure de phénylméthylsulfonyle et le benzamidine-HCI à la matrice osseuse pour décomposer le collagène contenu dans la matrice osseuse.

8. Procédé selon la revendication 7, comprenant en outre l'élimination du sel contenu dans la solution de réaction par dialyse après décomposition du collagène.
